Europäisches Patentamt

European Patent Office

Office européen des brevets

(19)

(11) Veröffentlichungsnummer : **0 081 647**
**B1**

(12) **EUROPÄISCHE PATENTSCHRIFT**

(45) Veröffentlichungstag der Patentschrift :
**09.01.85**

(21) Anmeldenummer : **82109381.2**

(22) Anmeldetag : **11.10.82**

(51) Int. Cl.⁴ : **C 07 C 37/16, C 07 C 39/07//**
**B01J23/86**

(54) **Verfahren zur ortho-Methylierung von Phenolverbindungen.**

(30) Priorität : **11.12.81 DE 3149022**

(43) Veröffentlichungstag der Anmeldung :
**22.06.83 Patentblatt 83/25**

(45) Bekanntmachung des Hinweises auf die Patenterteilung : **09.01.85 Patentblatt 85/02**

(84) Benannte Vertragsstaaten :
**BE DE FR GB IT NL SE**

(56) Entgegenhaltungen :
**EP-A- 0 050 937**
**DE-A- 1 948 607**
**GB-A- 1 124 839**
**CHEMICAL ABSTRACTS, Band 85, Nr. 7, 27. September 1976, Seite 606, Nr. 94053z, Columbus, Ohio, USA**

(73) Patentinhaber : **CHEMISCHE WERKE HÜLS AG - RSP Patente / PB 15 - Postfach 13 20 D-4370 Marl 1 (DE)**

(72) Erfinder : **Sigg, Reinhard, Dr. Am Alten Sportplatz 17a D-4370 Marl (DE)**

**0 081 647**

## Beschreibung

Die Erfindung betrifft ein Verfahren zur selektiven ortho-Methylierung von Phenolen, die in ortho-Stellung mindestens ein Wasserstoffatom besitzen, durch Umsetzung dieser Phenole mit Methanol in der Gasphase in Gegenwart eines eine Oxidmischung von Eisenoxid, Chromoxid und Ceroxid enthaltenden Katalysators bei Temperaturen von 270 bis 450 °C, einer Raumgeschwindigkeit von 0,05 bis 3 h$^{-1}$ (LHSV) und Drücken von 1 bar bis 40 bar absolut. Der Eisenoxid, Chromoxid, Ceroxid enthaltende Katalysator weist insbesondere eine hohe Selektivität und eine hohe Ausbeute zur ortho-Alkylierung sowie ein hohes Verhältnis von zweifach ortho-Alkylierung zur mono-ortho-Alkylierung während längerer Zeiträume auf.

Aus der DE-AS 21 27 083 (= GB-PS 1 323 211) ist es bekannt, daß Methanol mit Phenolen der oben angegebenen allgemeinen Struktur an einem Manganoxid und Ceroxid enthaltenden Katalysator bei Temperaturen von 300 bis 550 °C umgesetzt werden kann. Aus der britischen Patentschrift 717 588 ist bekannt, daß Methanol mit o-Kresol in der Gasphase in Gegenwart eines Katalysators reagiert, der nur aus einem der folgenden Metalloxide aufgebaut ist : Oxide von Magnesium, Aluminium, Calcium, Mangan, Eisen, Zink, Zirkonium, Barium, Thorium. In der britischen Patentschrift 1 124 839 ist ein Verfahren zur Herstellung von Alkylphenolen beschrieben, bei dem ein Phenol mit einem Alkohol in Gegenwart eines Oxids eines Elements der seltenen Erden, wie z. B. Cer, Lanthan, Neodym und Praseodym umgesetzt wird. Alle der in diesen Verfahren verwendeten Katalysatoren enthalten nur eine der aktiven Komponenten Eisenoxid bzw. Ceroxid.

Diese bekannten Katalysatoren besitzen mehrere Nachteile : Sie haben eine geringe Aktivität und eine geringe Selektivität für die ortho-Alkylierung. Zudem bewirken sie einen hohen Methanol-Verlust. Außerdem ist die Lebensdauer der Katalysatoren gering.

Es wurden weiterhin Katalysatoren aus Eisenoxid und Kieselsäure bzw. aus Eisenoxid, Kieselsäure und Chromoxid zur Herstellung von ortho-Alkylphenolen mit hoher Selektivität vorgeschlagen (DE-AS 24 28 056 = GB-PS 1 428 057), jedoch zeigt der binäre Katalysator ebenfalls eine starke Abnahme der Aktivität bei längerer Laufzeit. Der ternäre Katalysator hat zwar eine im Vergleich zum binären Katalysator verbesserte Lebensdauer, die mit einem quaternären Katalysator, bestehend aus Eisenoxid, Kieselsäure, Chromoxid und einer Alkalimetallverbindung bzw. Erdalkaliverbindung weiter verbessert werden kann (DE-PS 25 47 309 = GB-PS 1 507 478) und BE-PS 888 022 = GB-PS 2 072 674). Für den technischen Einsatz ist sie jedoch noch nicht ausreichend. Im Hinblick auf die technische Anwendung eines Katalysators ist es wichtig, eine hohe Selektivität und Ausbeute bei erheblich verbesserter Standzeit zu erzielen. Eine Aufgabe der vorliegenden Erfindung besteht in der Lösung dieses Problems.

Der Erfindung liegt die Aufgabe zugrunde, ein katalytisches Verfahren zur ortho-Methylierung von Phenolverbindungen, die mindestens ein Wasserstoffatom in ortho-Stellung besitzen, in der Gasphase zu entwickeln, das in einfacher Weise und in technischem Ausmaß bei gleichzeitig besserer Aktivität, Selektivität, Ausbeute und Standzeit des Katalysators durchgeführt werden kann.

Diese Aufgabe wird erfindungsgemäß entsprechend den Angaben der Ansprüche gelöst.

Der erfindungsgemäß verwendete ternäre Katalysator, der die kalzinierte ternäre oxidische Mischung aus Eisenoxid ($Fe_2O_3$, $Fe_3O_4$), Chromoxid ($Cr_2O_3$) und Ceroxid ($Ce_2O_3$) in den beanspruchten Molverhältnissen enthält, ist überraschenderweise wesentlich länger katalytisch aktiv im Vergleich zu den bekannten Katalysatoren, sogar im Vergleich zu den bekannten quaternären Katalysatoren. Der erfindungsgemäß verwendete ternäre Katalysator zeigt eine besonders hohe Selektivität zu 2.6-Dimethylphenol und o-Kresol sowie ein überraschend hohes Verhältnis der Ausbeute an 2.6-Dimethylphenol zu o-Kresol. Hierbei ist es besonders vorteilhaft, daß der Katalysator seine hohe Aktivität und Selektivität über einen Zeitraum von 3 000 Stunden und mehr behält. Innerhalb des Katalysatorbettes hält man die Temperatur in einem engen Temperaturbereich von $\Delta T$ bis 20 °C, bevorzugt bis 8 °C, insbesondere bis 5 °C weitgehend konstant.

Der erfindungsgemäße Katalysator besteht aus einer kalzinierten Mischung von Eisenoxid, Chromoxid und Ceroxid im Atomverhältnis Fe : Cr : Ce von 1 : 0,005 bis 0,1 : 0,005 bis 0,1, bevorzugt 1 : 0,005 bis 0,02 : 0,005 bis 0,02.

Als Phenolverbindungen sind beispielsweise Phenol, o-Kresol, p-Kresol und m-Kresol geeignet. Man setzt die Phenolverbindungen mit Methanol im Molverhältnis Phenol : Methanol von 1 : 1 bis 1 : 10, vorzugsweise von 1 : 2 bis 1 : 10, insbesondere von 1 : 3 bis 1 : 6 um.

Die Methylierung erfolgt in der Gasphase bei Temperaturen von 270 bis 450 °C, vorzugsweise von 290 bis 350 °C bei einem Druck von 1 bis 40 bar absolut, vorzugsweise von 3 bis 30 bar absolut, wobei man im unteren Temperaturbereich den niederen Druckbereich einstellt.

Das Reaktionsgemisch kann man mit Inertgasen wie Stickstoff verdünnen, so daß der Partialdruck des Gemisches aus Phenolverbindung und Methanol auch unter 1 bar absolut liegen kann. Die Umsetzung erfolgt bei Raumgeschwindigkeiten (LHSV), bezogen auf die flüssigen Einsatzkomponenten, von 0,05 h$^{-1}$ bis 3 h$^{-1}$.

Wird ein Phenol mit 2 Wasserstoffatomen in ortho-Stellung mit Methanol alkyliert, dann kann sowohl das entsprechende zweifach methylierte Phenol als auch das monomethylierte Phenol hergestellt werden. Bei dieser Reaktion werden bezüglich Phenol praktisch keine Nebenprodukte gebildet. Methanol kann am Katalysator neben der Alkylierung auch eine Zersetzungsreaktion zu primär Kohlenmonoxid und

2

Wasserstoff eingehen. Diese Zersetzungsprodukte reagieren weitgehend zu $CO_2$, $CH_4$ und $H_2O$ weiter.

Durch Auswahl der geeigneten Reaktionsbedingungen kann das Verfahren so gesteuert werden, daß es überwiegend zur Bildung eines zweifach methylierten Phenols oder überwiegend zur Bildung des monomethylierten Phenols führt. Auf diese Weise können bei Phenolen mit zwei Wasserstoffatomen in ortho-Stellung durch Erhöhung des Verhältnisses von Methanol zu Phenol im Edukt, durch Erhöhung der Reaktionstemperatur, durch Erhöhung des Reaktionsdruckes oder durch Erniedrigung der Raumgeschwindigkeit die Ausbeute und Selektivität an zweifach in ortho-Stellung methylierten Phenolen erheblich gesteigert werden. Durch die entgegengesetzten Maßnahmen erhält man bevorzugt in ortho-Stellung monomethylierte Phenole.

Zur Herstellung von o-Kresol setzt man bevorzugt die Phenolverbindung und das Methanol in Molverhältnissen von 1 : 1 bis 3 bei Temperaturen von 270 bis 350 °C, Drücken von 1 bis 4 bar und bei einer Raumgeschwindigkeit LHSV von 0,5 bis 3 h$^{-1}$ um. Zur Herstellung von 2,6-Dimethylphenol arbeitet man vorzugsweise bei einer Raumgeschwindigkeit LHSV von 0,05 bis 1 h$^{-1}$, Molverhältnissen Phenolverbindung zu Methanol von 1 : 3 bis 6, Temperaturen von 300 bis 450 °C und Drücken von 3 bis 30 bar.

Bei der Durchführung des Verfahrens der Erfindung kann jeder herkömmliche Reaktor für Reaktionen in der Gasphase verwendet werden. Vorteilhaft sind Reaktoren, bei denen die exotherme Wärmetönung der Gesamtreaktion sehr gut abgeführt wird und über die ganze Länge des Katalysatorbetts eine möglichst gleichbleibende Reaktorinnentemperatur eingehalten werden kann, wobei über das Katalysatorbett bis zu 20 °C Temperaturdifferenz, bevorzugt bis zu 8 °C, insbesondere bis 5 °C erreicht werden. Dieses erzielt man beispielsweise durch Verwendung eines Vielrohrreaktors mit guter Wärmeabfuhr und niedrigem Rohrinnendurchmesser, vorteilhaft bis zu 50 mm, oder durch Verdünnung des Katalysators mit katalytisch inertem Material oder durch Verdünnung des Eduktes mit inerten Gaskomponenten.

Als katalytisch inertes Material eignet sich beispielsweise Keramik, Glaskörper oder Korund. Man setzt das inerte Material in Mengen von 1 bis 30 Gewichtsprozent, bezogen auf den aktiven Katalysator, ein. Als inerte Gaskomponenten setzt man beispielsweise Stickstoff in Mengen von 0,1 bis 10 Volumenteilen pro Volumenteil gasförmiger Einsatzstoffe ein.

Das Verfahren kann beispielsweise so durchgeführt werden, daß ein Phenol, das mindestens ein Wasserstoff-Atom in ortho-Stellung besitzt, und Methanol getrennt oder in einer Mischung verdünnt oder in reiner Form in einen Vorerhitzer eingespeist werden, der mit einem Reaktor, vorteilhaft der oben beschriebenen Auslegung, verbunden ist und in dem sich der Katalysator verdünnt oder in reiner Form befindet. Dadurch werden die verdampften und vorerhitzten Reaktionsteilnehmer in reiner Form oder mit Stickstoff als inertem Trägergas kontinuierlich mit dem Katalysatorbett in Kontakt gebracht.

Das Katalysatorbett wird gegebenenfalls von außen geheizt, und durch die oben beschriebenen Maßnahmen die Reaktorinnentemperatur über das gesamte Katalysatorbett möglichst konstant gehalten. Durch diese Maßnahme sowie durch die Wahl der Katalysatorzusammensetzung der oben beschriebenen Art wird eine erhöhte Selektivität der Bildung von z. B. 2,6-Dimethylphenol oder wahlweise o-Kresol erreicht und gleichzeitig die Zersetzung des Methanols erheblich erniedrigt. Die im Reaktor gebildeten gasförmigen Reaktionsprodukte werden dann in einem Abscheider verflüssigt.

Die verflüssigten Reaktionsprodukte enthalten hohe Anteile an ortho-methylierten Produkten des Phenols und können durch verschiedene an sich bekannte Reinigungsverfahren, wie z. B. durch Destillation, Extraktion, Umkristallisation in reiner Form erhalten werden. Die optimale Reaktionstemperatur hängt von dem jeweils herzustellenden o-Methylierungsprodukt des Phenols, von der gewählten Raumgeschwindigkeit, vom gewählten Druck und von der Zusammensetzung des Katalysators ab. Sie liegen im Bereich von 270 bis 450 °C, vorzugsweise von 290 bis 350 °C. Die Raumgeschwindigkeit, bezogen auf die flüssigen Einsatzkomponenten, kann im Bereich von LHSV = 0,05 h$^{-1}$ bis 3 h$^{-1}$ variiert werden, vorzugsweise im Bereich von LHSV 0,08 h$^{-1}$ bis 1 h$^{-1}$. Der Druck ist in weitem Bereich von 1 bar bis 40 bar absolut wählbar.

Bevorzugt führt man die Reaktion unter einem Druck von 3 bis 30 Atmosphären durch, weil dann mit erhöhter Raumgeschwindigkeit gefahren werden kann, die Zersetzung des Methanols zu primär Kohlenmonoxid und Wasserstoff sowie die Weiterreaktion der Zersetzungsprodukte vermindert wird. Die Temperatur im Verdampfer ist an sich unkritisch, jedoch ist es von Vorteil, die Verdampfungstemperatur der Phenole dadurch zu erniedrigen, daß Methanol dampfförmig durch die Phenollösung durchgeleitet oder daß von vornherein ein Gemisch von Phenol mit Methanol verdampft wird.

Das molare Verhältnis von Methanol zu den entsprechenden Einsatzphenolen kann variieren, je nachdem, ob das Einsatzphenol in ortho-Stellung ein oder zwei Wasserstoffatome aufweist und je nachdem, ob das gewünschte Produkt einfach oder zweifach in ortho-Stellung methyliert werden soll. Das molare Verhältnis in der Eduktmischung liegt bei 1 bis 10 Mol des Methanols je Mol der Phenolverbindung, vorzugsweise bei 2 bis 10 Mol, insbesondere bei 3 bis 6 Mol. Nicht umgesetzte Methanol- und/oder Phenolverbindungen können nach Abtrennung aus dem Reaktionsprodukt dem Edukt wieder zugemischt werden.

Der erfindungsgemäß verwendete Katalysator kann beispielsweise nach bekannten Verfahren als Fällungskatalysator oder durch innige Vermischung der Oxide von Eisen ($Fe_2O_3$), Chrom ($Cr_2O_3$) und Cer ($Ce_2O_3$) oder durch eine Kombination beider Verfahren oder durch Imprägnierung von $Fe_2O_3$ mit

Chrom und Cer hergestellt werden. Von den aufgeführten Verfahren wird das Fällungsverfahren besonders bevorzugt.

Beispielsweise kann man eine wäßrige Lösung der Nitrate, Chloride oder Sulphate des dreiwertigen Eisens, Chroms und Cers mit einem alkalischen Medium ausfällen, die überstehende Lösung abfiltrieren und den gewaschenen Niederschlag trocknen. Der getrocknete, in eine Form, wie z. B. Tabletten, Pellets oder Strangpreßlinge, gebrachte Niederschlag wird sodann 5 bis 10 Stunden bei 300 bis 500 °C kalziniert. Das atomare Verhältnis von Eisen zu Chrom zu Cer beträgt 1 : 0,005 bis 0,1 : 0,005 bis 0,1.

Die nach dem Verfahren der Erfindung in ortho-Stellung methylierten Produkte des Phenols können als Ausgangsmaterialien für die Synthese von Herbiciden, Antioxidantien, antiseptischen Mitteln, Vitamin E dienen. Das 2,6-Dimethylphenol setzt man insbesondere als Ausgangsmaterial für die Herstellung von Polyphenylenoxid ein. Das Verfahren der Erfindung wird anhand der folgenden Beispiele im einzelnen erläutert.

## Vergleichsbeispiel 1

Zur Herstellung des Katalysators löst man 300 g Eisen (III)-nitrat in 3 l destilliertem Wasser. Der Lösung gibt man zunächst 1,6 g Wasserglas zu. Dann fällt man aus der Lösung unter Rühren mit 10 %iger Ammoniak-Lösung das dreiwertige Eisen vollständig aus. Nach Waschen mit Wasser und Trocknen des filtrierten Niederschlags behandelt man das Katalysatorpulver bei Raumtemperatur in verdünnter wäßriger Kaliumcarbonatlösung nach. Den erneut filtrierten, getrockneten Niederschlag verpreßt man zu Tabletten und kalziniert 7 h bei 470 °C. Das Gewicht der Katalysatorcharge beträgt 43 g. 33,2 g des Katalysators werden in einen Reaktor mit 22 mm Innendurchmesser gefüllt und durch ein Salzbad temperaturgeregelt. Mit einer kontinuierlich betriebenen Flüssigdosierpumpe wird ein Methanol/Phenol-Gemisch mit dem Molverhältnis 5/1 Mol Methanol/Mol Phenol und einer Raumgeschwindigkeit von LHSV = 0,68 h$^{-1}$ in einem Verdampfer bei 140 °C verdampft und auf 300 °C vorgeheizt. Dem Gasgemisch wird vor Eintritt in den Reaktor Stickstoff als Trägergas zugeführt. Der genau eingestellte Stickstoffgehalt dient gleichzeitig als innerer Standard für die gaschromatographische Analyse der Zersetzungsprodukte des Methanols. Bei einer Reaktionstemperatur von 337 °C ergibt sich ein Phenol-Umsatz von 95 % bei 57 % Ausbeute an 2,6-Dimethylphenol und 35 % Ausbeute an o-Kresol. Diese Ausbeuten lassen sich nur innerhalb eines Zeitraumes von 50 Stunden erzielen. Anschließend nehmen die Aktivität und Selektivität der Bildung von 2,6-Dimethylphenol stark ab. Außerdem werden in geringen Mengen Anisol, 2,5-Dimethylphenol, 3,6-Dimethylphenol und 2,4-Dimethylphenol nachgewiesen. Die Zersetzung des Methanols liefert 24 mmol/h $CO_2$, 15 mmol/h $CH_4$, 5 mmol/h CO. Außerdem bildet sich Wasserstoff.

## Vergleichsbeispiel 2

300 g Eisen (III)-nitrat und 3 g Chrom (III)-nitrat werden in 3 l destilliertem Wasser gelöst und sodann aus der Lösung unter ständigem Rühren mit 10 %iger Ammoniaklösung das Eisen und das Chrom vollständig ausgefällt. Nach Waschen mit Wasser und Trocknen des filtrierten Niederschlags wird das Katalysatorpulver in verdünnter wäßriger Kaliumcarbonat-Lösung nachbehandelt. Der erneut filtrierte, getrocknete Niederschlag wird zu Tabletten verpreßt und 7 h bei 470 °C kalziniert getempert. Der Katalysator hat ein Atomverhältnis Eisen zu Chrom von 1 : 0,01. 29,5 g des Katalysators werden mit einem 5/1 Mol Methanol/Mol Phenol-Gemisch mit einer LHSV von 0,68 h$^{-1}$ beschickt. Das nach seiner Verdampfung bei 140 °C mit $N_2$ als Trägergas verdünnte Gasgemisch wird bei 372 °C zu 83 % umgesetzt. Die Ausbeute beträgt 34 % 2,6-Dimethylphenol und 47 % o-Kresol. Nach einer Laufzeit von 40 Stunden nimmt die Aktivität und Selektivität des Katalysators stark ab.

## Vergleichsbeispiele 3 bis 6

300 g Eisen (III)-nitrat, 3 g Chrom (III)-nitrat und 1,6 g Wasserglas, in 3 l destilliertem Wasser gelöst, werden mit 10 %iger Ammoniaklösung ausgefällt, gewaschen, filtriert, bei 180 °C getrocknet zu Tabletten verpreßt und 7 h bei 470 °C im Luftstrom kalziniert getempert.

Der Katalysator, der Eisen, Chrom und Silizium im Atomverhältnis 1 : 0,01 : 0,01 enthält, liefert bei der Methylierung eines 5 : 1 Mol Methanol/Mol Phenol-Gemisches, das nach Verdampfung bei 140 °C mit $N_2$ als Trägergas in den Reaktor gelangte, folgende Ergebnisse :

| Nr. | LHSV h$^{-1}$ | $\Delta T$ °C | $T_{Reaktor}$ °C | $U_{Phenol}$ % | $A_{o\text{-}Kresol}$ % | $A_{2,6\text{-}DMP}$ % | $S_{2,6\ DMP}$ % |
|---|---|---|---|---|---|---|---|
| 3 | 0,68 | 12 | 325 | 90 | 55 | 33 | 39 |
| 4 | 0,47 | 15 | 334 | 94 | 50 | 42 | 46 |
| 5 | 0,47 | 15 | 327 | 92 | 36 | 48 | 52 |
| 6 | 0,47 | 18 | 350 | > 99 | 4 | 90 | 90 |

Nach einer Laufzeit von 400 Stunden nimmt die Aktivität und Selektivität des Katalysators stark ab.

## Beispiele 7 und 8

900 g Eisen (III)-nitrat, 4,5 g Chrom (III)-nitrat und 4,8 g Cer (III)-nitrat werden in 9 l destilliertem Wasser gelöst und mit 10 %iger Ammoniaklösung gefällt, anschließend gewaschen, filtriert, bei 170 °C getrocknet, zu Tabletten verpreßt und 7 h bei 470 °C im Luftstrom kalziniert. Der Katalysator weist ein Atomverhältnis von Fe zu Cr zu Ce von 1 : 0,005 : 0,005 auf.

Bei der Methylierung eines 5 : 1 Mol Methanol/Mol Phenol-Gemisches, das nach Verdampfung bei 220 °C in den Reaktor gelangte, erhält man folgende Ergebnisse :

| Nr. | LHSV $h^{-1}$ | $T_{Reakt.}$ °C | $\Delta T$ °C | $U_{Phenol}$ % | $A_{o\text{-}Kresol}$ % | $A_{2,6\text{-}DMP}$ % |
|---|---|---|---|---|---|---|
| 7 | 0,13 | 314 | 4 | > 99 | 11 | 86 |
| 8,1 | 0,08 | 316 | 4 | > 99 | 4 | 94 |
| 2 | 0,08 | 317,5 | 5 | > 99 | 8 | 90 |
| 3 | 0,08 | 318 | 5 | > 99 | 6,5 | 91 |
| 4 | 0,08 | 318,5 | 2,5 | > 99 | 8,2 | 89,5 |
| 5 | 0,08 | 320 | 3 | > 99 | 0,3 | 96,4 |

| Nr. | $CH_3OH$-Verlust durch Zersetzung zu $CO_2$, $CH_4$, CO u. $H_2$ % | Betriebszeit h |
|---|---|---|
| 7 | 24 | 120 |
| 8,1 | 25,5 | 860 |
| 2 | 27,1 | 1 340 |
| 3 | 30 | 1 500 |
| 4 | 30 | 2 000 |
| 5 | 33 | 3 000 |

Innerhalb der untersuchten Betriebszeit bleiben Umsatz und Ausbeute praktisch konstant.

## Beispiele 9 bis 16

1,8 kg Eisen (III)-nitrat, 18 g Chrom (III)-nitrat und 19,3 g Cer (III)-nitrat, in 18 l Wasser gelöst, werden mit 10 %iger Ammoniaklösung gefällt, filtriert, gewaschen, bei 170 °C getrocknet, zu Pillen verpreßt und 7 h bei 470 °C im Luftstrom getempert. Das Atomverhältnis von Fe zu Cr zu Ce im Katalysator beträgt 1 : 0,01 : 0,01.

Bei der Methylierung mit einem 6 : 1 Mol Methanol/Mol Phenol-Gemisch erhält man folgende Ergebnisse :

| Nr. | LHSV $h^{-1}$ | $T_{Reakt.}$ °C | $\Delta T$ °C | $P_E$ bar | $U_{Phenol}$ % | $A_{o\text{-}Kresol}$ % | $A_{2,6\text{-}DMP}$ % | Betriebszeit h |
|---|---|---|---|---|---|---|---|---|
| 9 | 0,37 | 376 | 12 | 3,3 | 94 | 53 | 36 | 280 |
| | 0,37 | 380 | 12 | 3,3 | 92 | 59 | 32 | 550 |
| 10 | 0,37 | 377 | 12 | 4,4 | 94 | 53 | 38 | 600 |
| 11 | 0,37 | 380 | 13 | 5,5 | 95 | 45 | 46 | 700 |
| 12 | 0,24 | 380 | 13 | 1,7 | 89 | 60 | 26 | 1 000 |
| 13 | 0,24 | 380 | 13 | 10 | 90 | 40 | 46 | 750 |
| 14 | 0,24 | 380 | 12 | 30 | 95 | 33 | 59 | 750 |
| 15 | 0,24 | 380 | 8 | 30 | 90 | 31 | 55 | 1 500 |
| 16 | 0,24 | 400 | 8 | 35 | 90 | 25 | 61 | 3 000 |

Innerhalb der untersuchten Betriebszeit bleiben Umsatz und Ausbeute praktisch konstant.

Man erhält gleiche Ergebnisse, wenn man einen Katalysator einsetzt, der 10 Gewichtsprozent Glasperlen mit einem Durchmesser von 2 bis 4 mm, bezogen auf den in diesem Beispiel eingesetzten Katalysator, enthält.

## Beispiel 17

43 g des Katalysators aus Beispiel 7 und 8 werden in einem Reaktor mit 22 mm Innendurchmesser gefüllt und durch ein Salzbad temperaturgeregelt. Ein o-Kresol/Methanol-Gemisch mit dem Molverhältnis 3 Mol Methanol/1 Mol o-Kresol wird mit einer Raumgeschwindigkeit von LHSV = 0,5 h$^{-1}$ und einer Reaktorinnentemperatur von 335 °C ($\Delta$T 8 °C) bei 3 bar umgesetzt. Der o-Kresol-Umsatz beträgt > 99 %, die Ausbeute an 2,6-Dimethylphenol beträgt 98 %.

## Beispiel 18

43 g des Katalysators aus Beispiel 7 und 8 werden nach den Angaben des Beispiels 17, jedoch mit einem p-Kresol/Methanol-Gemisch mit dem Molverhältnis 5 Mol Methanol/1 Mol p-Kresol umgesetzt. Bei praktisch vollständigem Umsatz an p-Kresol (> 99 %) beträgt die Ausbeute an 2,4-Dimethylphenol 36 % und die Ausbeute an 2,4,6-Trimethylphenol 59 %.

## Beispiel 19

43 g des Katalysators aus Beispiel 17 werden nach den Angaben dieses Beispiels, jedoch mit einem m-Kresol/Methanol-Gemisch mit dem Molverhältnis 5 Mol Methanol/1 Mol m-Kresol umgesetzt.

U (m-Kresol) > 99 %
A (2,3-Dimethylphenol) = 45 %
A (2,3,6-Trimethylphenol) = 46 %.

**Ansprüche** (für die Vertragsstaaten : BE, DE, FR, GB)

1. Verfahren zur ortho-Methylierung von Phenolverbindungen, die mindestens ein Wasserstoffatom in ortho-Stellung besitzen, mit Methanol in einem Molverhältnis Phenolverbindung zu Methanol von 1 : 1 bis 1 : 10 in der Gasphase in Gegenwart eines Metalloxid-Katalysators, der eine kalzinierte Mischung aus den Oxiden von Eisen, Chrom und Cer im Atomverhältnis von 1 : 0,005 bis 0,1 : 0,005 bis 0,1 enthält, bei Temperaturen von 270 bis 450 °C, einem Druck von 1 bis 40 bar absolut und Raumgeschwindigkeiten (LHSV) von 0,05 bis 3 h$^{-1}$ zu o-Kresol, 2,6-Dimethylphenol, 2,3- bzw. 2,4-Dimethylphenol und/oder 2,3,6- bzw. 2,4,6-Trimethylphenol, dadurch gekennzeichnet, daß man innerhalb des Katalysatorbettes die Temperatur in einem Temperaturbereich von $\Delta$T bis 20 °C weitgehend konstant hält.

2. Verfahren nach Anspruch 1, dadurch gekennzeichnet, daß man innerhalb des Katalysatorbettes die Temperatur in einem Bereich von $\Delta$T bis 8 °C, vorzugsweise in einem Bereich von $\Delta$T bis 5 °C weitgehend konstant hält.

3. Verfahren nach Anspruch 1 und 2, dadurch gekennzeichnet, daß man zur Herstellung von o-Kresol bei einer Raumgeschwindigkeit LHSV von 0,5 bis 3 h$^{-1}$, Molverhältnissen von Phenol zu Methanol von 1 : 1 bis 3, Temperaturen von 270 bis 350 °C und Drücken von 1 bis 4 bar arbeitet.

4. Verfahren nach Anspruch 1 und 2, dadurch gekennzeichnet, daß man zur Herstellung von 2,6-Dimethylphenol bei einer Raumgeschwindigkeit LHSV von 0,05 bis 1 h$^{-1}$, Molverhältnissen Phenolverbindung zu Methanol von 1 : 3 bis 6, Temperaturen von 300 bis 450 °C und Drücken von 3 bis 30 bar arbeitet.

**Ansprüche** (für die Vertragsstaaten : IT, NL, SE)

1. Verfahren zur ortho-Methylierung von Phenolverbindungen, die mindestens ein Wasserstoffatom in ortho-Stellung besitzen, mit Methanol in einem Molverhältnis Phenolverbindung zu Methanol von 1 : 1 bis 1 : 10 in der Gasphase in Gegenwart eines Metalloxid-Katalysators bei Temperaturen von 270 bis 450 °C, einem Druck von 1 bis 40 bar absolut und Raumgeschwindigkeiten (LHSV) von 0,05 bis 3 h$^{-1}$ zu o-Kresol, 2,6-Dimethylphenol, 2,3- bzw. 2,4-Dimethylphenol und/oder 2,3,6- bzw. 2,4,6-Trimethylphenol, dadurch gekennzeichnet, daß man einen Metalloxid-Katalysator einsetzt, der eine kalzinierte Mischung aus den Oxiden von Eisen, Chrom und Cer im Atomverhältnis 1 : 0,005 bis 0,1 : 0,005 bis 0,1 enthält, und man innerhalb des Katalysatorbettes die Temperatur in einem Temperaturbereich von $\Delta$T bis 20 °C weitgehend konstant hält.

2. Verfahren nach Anspruch 1, dadurch gekennzeichnet, daß man einen Metalloxid-Katalysator einsetzt, der Eisen, Chrom und Cer im Atomverhältnis von 1 : 0,005 bis 0,02 : 0,005 bis 0,02 enthält.

3. Verfahren nach Anspruch 1 und 2, dadurch gekennzeichnet, daß man innerhalb des Katalysatorbettes die Temperatur in einem Bereich von $\Delta$T bis 8 °C, vorzugsweise in einem Bereich von $\Delta$T bis 5 °C weitgehend konstant hält.

4. Verfahren nach Anspruch 1 bis 3, dadurch gekennzeichnet, daß man zur Herstellung von o-Kresol bei einer Raumgeschwindigkeit LHSV von 0,5 bis 3 h$^{-1}$, Molverhältnissen von Phenol zu Methanol von 1 : 1 bis 3, Temperaturen von 270 bis 350 °C und Drücken von 1 bis 4 bar arbeitet.

5. Verfahren nach Anspruch 1 bis 3, dadurch gekennzeichnet, daß man zur Herstellung von 2,6-Dimethylphenol bei einer Raumgeschwindigkeit LHSV von 0,05 bis 1 $h^{-1}$, Molverhältnissen Phenolverbindung zu Methanol von 1 : 3 bis 6, Temperaturen von 300 bis 450 °C und Drücken von 3 bis 30 bar arbeitet.

**Claims** (for the Contracting States : BE, DE, FR, GB)

1. A process for the ortho-methylation of a phenolic compound which possesses at least one hydrogen atom in an ortho-position by means of methanol, using a molar ratio of phenolic compound to methanol of from 1 : 1 to 1 : 10, in the gas phase in the presence of a metal oxide catalyst which contains a calcined mixture of the oxides of iron, chromium and cerium in an atomic ratio of 1 : 0.005-0.1 : 0.005-0.1, at a temperature of 270 to 450 °C under a pressure of 1 to 40 bar absolute and at a liquid hourly space velocity of 0.05 to 3 $h^{-1}$, to give o-cresol, 2,6-dimethylphenol, 2,3- and 2,4-dimethylphenol and/or 2,3,6- and 2,4,6-trimethylphenol, characterised in that within the catalyst bed the temperature is kept substantially constant within a temperature range ($\Delta T$) of up to 20 °C.

2. A process according to claim 1, characterised in that within the catalyst bed the temperature is kept substantially constant within a range ($\Delta T$) of up to 8 °C, preferably with a range ($\Delta T$) of up to 5 °C.

3. A process according to claim 1 or 2, characterised in that, to produce o-cresol, the process is carried out at a liquid hourly space velocity of 0.5 to 3 $h^{-1}$, a molar ratio of phenol to methanol of 1 : 1-3, a temperature of 270 to 350 °C and a pressure of 1 to 4 bar.

4. A process according to claim 1 or 2, characterised in that to produce 2,6-dimethylphenol, the process is carried out at a liquid hourly space velocity of 0.05 to 1 $h^{-1}$, a molar ratio of phenolic compound to methanol of 1 : 3-6, a temperature of 300 to 450 °C and a pressure of 3 to 30 bar.

**Claims** (for the Contracting States : IT, NL, SE)

1. A process for the ortho-methylation of a phenolic compound which possesses at least one hydrogen atom in an ortho-position by means of methanol, using a molar ratio of phenolic compound to methanol of from 1 : 1 to 1 : 10, in the gas phase in the presence of a metal oxide catalyst at a temperature of 270 to 450 °C under a pressure of 1 to 40 bar absolute and at a liquid hourly space velocity of 0.05 to 3 $h^{-1}$, to give o-cresol, 2,6-di-methylphenol, 2,3- and 2,4-dimethylphenol and/or 2,3,6- and 2,4,6-trimethylphenol, characterised in that a metal oxide catalyst is used which contains a calcined mixture of the oxides of iron, chromium and cerium in an atomic ratio of 1 : 0.005-0.1 : 0.005-0.1 and that the temperature within the catalyst bed is kept substantially constant within a temperature range ($\Delta T$) of up to 20 °C.

2. A process according to claim 1, characterised in that a metal oxide catalyst which contains iron, chromium and cerium in an atomic ratio of 1 : 0.005-0.02 : 0.005-0.02 is used.

3. A process according to claim 1 or 2, characterised in that within the catalyst bed the temperature is kept substantially constant within a range ($\Delta T$) of up to 8 °C, preferably with a range ($\Delta T$) of up to 5 °C.

4. A process according to any of claims 1 to 3, characterised in that, to produce o-cresol, the process is carried out at a liquid hourly space velocity of 0.5 to 3 $h^{-1}$, a molar ratio of phenol to methanol of 1 : 1-3, a temperature of 270 to 350 °C and a pressure of 1 to 4 bar.

5. A process according to any of claims 1 to 3, characterised in that to produce 2,6-dimethylphenol, the process is carried out at a liquid hourly space velocity of 0.05 to 1 $h^{-1}$, a molar ratio of phenolic compound to methanol of 1 : 3-6, a temperature of 300 to 450 °C and a pressure of 3 to 30 bar.

**Revendications** (pour les Etats contractants : BE, DE, FR, GB)

1. Procédé d'ortho-méthylation de composés phénoliques qui présentent au moins un atome d'hydrogène en position ortho, au moyen de méthanol dans un rapport molaire composé phénolique/méthanol compris entre 1 : 1 et 1 : 10, en phase gazeuse, en présence d'un catalyseur à oxyde métallique qui contient un mélange calciné des oxydes de fer, de chrome et de cérium dans un rapport atomique de 1 : (0,005 à 0,1) : (0,005 à 0,1), à des températures de 270 à 450 °C, à une pression absolue de 1 à 40 bar et à des vitesses spatiales de 0,05 à 3 $h^{-1}$ avec obtention d'ortho-crésol, de 2,6-diméthylphénol, de 2,3- ou 2,4-diméthylphénol et/ou de 2,3,6 ou 2,4,6-triméthylphénol, caractérisé par le fait qu'au sein de la couche du catalyseur, on maintient la température constante dans une large mesure, dans un intervalle de température de $\Delta T$ à 20 °C.

2. Procédé selon la revendication 1, caractérisé par le fait qu'au sein de la couche du catalyseur, on maintient la température constante dans une large mesure, dans un intervalle de $\Delta T$ à 8 °C, de préférence dans un intervalle de $\Delta T$ à 5 °C.

3. Procédé selon les revendications 1 et 2, caractérisé par le fait que pour la préparation d'ortho-crésol, on opère à une vitesse spatiale de 0,5 à 3 $h^{-1}$, à des rapports molaires phénol/méthanol de 1 : (1 à

3), à des températures de 270 à 350 °C et à des pressions de 1 à 4 bar.

4. Procédé selon les revendications 1 et 2, caractérisé par le fait que, pour la préparation de 2,6-diméthylphénol, on opère à une vitesse spatiale de 0,05 à 1 h$^{-1}$, à des rapports molaires composé phénolique/méthanol de 1 : (3 à 6), à des températures de 300 à 450 °C et à des pressions de 3 à 30 bar.

**Revendications** (pour les Etats contractants : IT, NL, SE)

1. Procédé d'ortho-méthylation de composés phénoliques présentant au moins un atome d'hydrogène en position ortho, au moyen de méthanol dans un rapport molaire composé phénolique/méthanol compris entre 1 : 1 à 1 : 10, en phase gazeuse, en présence d'un catalyseur à base d'oxyde métallique, à des températures de 270 à 450 °C, sous une pression absolue de 1 à 40 bar et à des vitesses spatiales de 0,05 à 3 h$^{-1}$, avec obtention d'ortho-crésol, de 2,6-diméthylphénol, de 2,3- ou de 2,4-diméthylphénol et/ou de 2,3,6- ou 2,4,6-triméthylphénol, caractérisé par le fait qu'on utilise un catalyseur à oxyde métallique qui contient un mélange calciné des oxydes de fer, de chrome et de cérium dans un rapport atomique de 1 : (0,005 à 0,1) : (0,005 à 0,1) et qu'au sein de la couche du catalyseur, on maintient la température constante dans une large mesure, dans un intervalle de température de ΔT à 20 °C.

2. Procédé selon la revendication 1, caractérisé par le fait qu'on utilise un catalyseur à base d'oxyde métallique, qui renferme du fer, du chrome et du cérium dans un rapport atomique de 1 : (0,005 à 0,02) : (0,005 à 0,02).

3. Procédé selon les revendications 1 et 2, caractérisé par le fait qu'au sein de la couche de catalyseur, on maintient la température constante dans une large mesure, dans un intervalle de température de ΔT à 8 °C, de préférence dans un intervalle de température de ΔT à 5 °C.

4. Procédé selon les revendications 1 à 3, caractérisé par le fait que, pour la préparation d'ortho-crésol, on travaille à une vitesse spatiale de 0,5 à 3 h$^{-1}$, à des rapports molaires composé phénolique/méthanol de 1 : (1 à 3), à des températures de 270 à 350 °C et sous des pressions de 1 à 4 bar.

5. Procédé selon les revendications 1 à 3, caractérisé par le fait que, pour la préparation de 2,6-diméthylphénol, on opère à une vitesse spatiale de 0,05 à 1 h$^{-1}$, à des rapports molaires composé phénolique/méthanol de 1 : (3 à 6), à des températures de 300 à 450 °C et sous des pressions de 3 à 30 bar.